# EUROPÄISCHE PATENTSCHRIFT

(11) **EP 2 240 610 B1**
(45) Veröffentlichungstag und Bekanntmachung des Hinweises auf die Patenterteilung: **29.08.2012**
(21) Anmeldenummer: 09707581.6
(22) Anmeldetag: 06.02.2009
(51) Int. Cl.: C12Q 1/68

(54) **AMPLIFIKATION VON BISULFITIERTEN NUKLEINSÄUREN**
AMPLIFICATION OF BISULFITE-REACTED NUCLEIC ACIDS
AMPLIFICATION D'ACIDES NUCLÉIQUES BISULFITÉS

(30) Priorität: 07.02.2008 DE 102008008313
(43) Veröffentlichungstag der Anmeldung: 20.10.2010
(73) Patentinhaber: Qiagen GmbH, 40724 Hilden (DE)
(72) Erfinder: KORFHAGE, Christian, 40724 Hilden (DE); LOEFFERT, Dirk, 40724 Hilden (DE); PEIST, Ralf, 40724 Hilden (DE); RUDINGER, Nicolas, 40724 Hilden (DE)
(74) Vertreter: Michalski Hüttermann & Partner
(86) Internationale Anmeldenummer: PCT/EP2009/051388
(87) Internationale Veröffentlichungsnummer: WO 2009/098298

(56) Entgegenhaltungen:
- WO-A-00/70090
- WO-A-2004/067775
- OLEK A ET AL: "A modified an improved method for bisulphite based cytosine methylation analysis" NUCLEIC ACIDS RESEARCH, OXFORD UNIVERSITY PRESS, SURREY, GB, Bd. 24, Nr. 24, 1. Januar 1996 (1996-01-01), Seiten 5064-5066, XP002106408 ISSN: 0305-1048

## Beschreibung

### Technisches Gebiet

Die vorliegende Erfindung liegt auf dem Gebiet der Molekularbiologie und Epigenetik und betrifft die Amplifikation von Nukleinsäuren, deren unmethylierte Cytosinbasen mittels einer Bisulfitierungsreaktion in Uracilbasen umgewandelt wurden. Des Weiteren werden Reaktionsgemische und Kits für die erfindungsgemäße Nukleinsäureamplifikation, sowie Verfahren zur Analyse des Methylierungszustands von Nukleinsäure offenbart.

### Stand der Technik

Epigenetische Mechanismen verursachen Änderungen der Genexpression, die nicht mit einer Veränderung der kodierenden Sequenz der Gene einhergehen, jedoch z.B. mitotisch vererbt werden können. In höheren Eukaryoten stellt neben RNA-assoziiertem Silencing und Histonmodifikation die DNA-Methylierung den am besten untersuchten epigenetischen Mechanismus dar (Serman et al., Coll Antropol. 2006; 30(3):665-71).

Die DNA-Methylierung findet an den Cytosinresten der Nukleinsäure und vorzugsweise bei Dinukleotiden mit einer Cytosin-Guanin-Sequenz (CpG) statt. Die wichtigste Basenmodifizierung bei Eukaryoten ist die Methylierung an der 5'-Position des Cytosin ,

Die Art der Methylierung kann jedoch unterschiedlich sein , neben der CpG-Methylierung wurde bei Pflanzen CNG sowie asymmetrische Cytosin-Methylierung bekannt, bei Drosophila wurde CT-Methylierung vom geringen Ausmaß nachgewiesen. Weitherhin wurde eine nicht-CG-Methylierung bei verschiedenen Pilzarten gefunden. Die CpG-Dinukleotide treten oft in kleineren (500 bp - 4 kb) Abschnitten der Nukleinsäure angehäuft auf und werden bei Vorliegen eines GC-Gehalt von > 55% als CpG-Islands bezeichnet. CpG-Islands finden sich für gewöhnlich im Bereich regulatorischer Regionen von Genen und können die Regulation der Transkription dieser Gene beeinflussen. Die Methylierung der CpG- Islands führt meist zu Inaktivierung des betreffenden Gens und spielt damit bei einer Reihe biologischer Prozesse (Imprinting, X-chromosomaler Inaktivierung, Genexpression), jedoch insbesondere bei der Entstehung von gutartigen als auch bösartigen Tumoren (Egger et al., Nature. 2004; 429(6990):457-63), bei einigen Phänotypen, Krankheiten und bei der Zellalterung eine bedeutende Rolle.

Die Analyse der Nukleinsäure-Methylierung ist daher insbesondere in der Diagnostik von Bedeutung (z.B. für die Früherkennung und Klassifizierung von Krebs und anderen Krankheiten), während die gerichtete Änderung des Methylierungszustands zum Zwecke der Genregulation eine potentielle therapeutische Strategie darstellt. Daher nimmt die Detektion des Methylierungszustands von Nukleinsäure einen hohen Stellenwert ein.

Zur Analyse des Methylierungszustands von Nukleinsäure kann diese bisulfitiert werden, wobei unmethylierte Cytosinbasen der Nukleinsäure in Uracilbasen umgewandelt werden, während methylierte Cytosinbasen unverändert bleiben. Eine Übersicht über verschiedene Techniken zur Analyse des Methylierungszustands von Nukleinsäuren, insbesondere die Bisulfitierung, findet sich in Fraga et al., Biotechniques. 2002; 33(3):632, 634, 636-49 und Laird, Nat Rev Cancer. 2003;3(4):253-66. Die Bisulfitierungreaktion führt daher abhängig vom Methylierungszustand der Ausgangsnukleinsäure zu Nukleinsäuresequenzen mit unterschiedlicher Sequenz, nach deren Analyse, unter anderem durch PCR oder Sequenzierung, auf den Methylierungszustand der Ausgangsnukleinsäure zurückgeschlossen werden kann.

In Abhängigkeit vom Entwicklungszustand und vom physiologischem Zustand der Zelle liegen ca. 5-15% aller Cytosinbasen methyliert vor. Da die methylierten Cytosinbasen von der Bisulfitierungsreaktion unbetroffen bleiben, führt dies dazu, dass ca. 85-95% aller Cytosinbasen der Ausgangsnukleinsäure in Uracilbasen umgewandelt werden. Folglich erhält hierdurch die Ausgangsnukleinsäure eine weitgehend neue Basenzusammensetzung, wobei aufgrund der Umwandlung von Cytosin in Uracil aus G:C-Basenpaaren G:U-Misspaarungen entstehenden und somit die beiden Stränge der Nukleinsäure fortan nicht mehr vollständig komplementär zueinander sind. Die erzeugten Misspaarungen können erheblich sein, da z.B. eine DNA, die 5% methyliertes Cytosin bei einem GC Gehalt von 50% enthält, nach der Bisulfitierung einen GC Gehalt von nur noch 28% aufweisen kann.

Die Amplifikation dieser nach der Bisulfitierungsreaktion erhaltenen umgewandelten Nukleinsäure mit veränderten Basenzusammensetzung kann, insbesondere bei der PCR-Amplifikation, zu großen Schwierigkeiten führen. Daher besteht ein Bedarf an einer verbesserten Amplifikation von Nukleinsäuren, die durch Bisulfitierung umgewandelt wurden. Die vorliegende Erfindung stellt Lösungen für dieses Problem bereit.

### Zusammenfassung der Erfindung

Gegenstand der vorliegende Erfindung sind verbesserte Reaktionsgemische und Verfahren für die Amplifikation mittels Bisulfitierung umgewandelter Nukleinsäure, Verfahren zur Analyse des Methylierungszustands von Nukleinsäure, sowie korrespondierende Kits. Die vorliegende Erfindung basiert auf der überraschenden Entdeckung, dass die Amplifikation von Nukleinsäure, die einer Umwandlung durch eine Bisulfitierungsreaktion unterzogen wurde, durch eine Erhöhung der Konzentration der Nukleotide dATP und dTTP im Reaktionsgemisch gegenüber der Konzentration von dCTP und dGTP signifikant verbessert werden kann.

Ein Aspekt der vorliegenden Erfindung betrifft ein Reaktionsgemisch für die Nukleinsäureamplifikation umfassend dATP, dTTP, dCTP und dGTP, sowie Nukleinsäure enthaltend Uracil dadurch gekennzeichnet, dass die Anfangskonzentration von dATP und dTTP höher als die von dCTP und dGTP ist.

Besonders bevorzugt ist dabei vorgesehen, dass die verwendeten Nukleotide nicht markiert bzw. gelabelt sind, also insbesondere keine Fluoreszenz-, Lumineszenz-, oder Gewichtsmarker, farbstoffhaltige oder eine Färbung erzeugende Marker (insbesondere Enzymmarker), magnetische, radioaktive, radiologisch nachweisbare oder immunologische Marker, Antigene, Lektine oder sonstwie detektierbare Marker oder Labels aufweisen.

In einer bevorzugten Ausführungsform liegt die Anfangskonzentration von dATP und dTTP im Reaktionsgemisch bei 200% bis 600% der Anfangskonzentration von dCTP und dGTP. In einer weiteren bevorzugten Ausführungsform liegt die Anfangskonzentration von dATP und dTTP im Reaktionsgemisch bei 300% bis 500% der Anfangskonzentration von dCTP und dGTP.

Ein zweiter Aspekt der vorliegenden Erfindung betrifft ein Verfahren zur Amplifikation von mittels einer Bisulfitierungsreaktion umgewandelten Nukleinsäure umfassend die Schritte:
a. In Kontakt bringen der Nukleinsäure mit zumindest einem Primersatz, zumindest einer Polymerase, sowie dATP, dTTP, dCTP und dGTP; und
b. Amplifikation der Nukleinsäure mittels der Polymerase,
dadurch gekennzeichnet, dass die Anfangskonzentration von dATP und dTTP höher als die von dCTP und dGTP ist. In einer bevorzugten Ausführungsform liegt die Anfangskonzentration von dATP und dTTP im Reaktionsgemisch bei 200% bis 600% der Anfangskonzentration von dCTP und dGTP. In einer weiteren bevorzugten Ausführungsform liegt die Anfangskonzentration von dATP und dTTP im Reaktionsgemisch bei 300% bis 500% der Anfangskonzentration von dCTP und dGTP. In einer weiteren bevorzugten Ausführungsform wird die Amplifikation isothermal oder mittels Temperatur-Cycling durchgeführt, noch bevorzugter ist die Amplifikation eine RCA oder eine MDA.

Ein weiterer Aspekt der vorliegenden Erfindung betrifft ein Verfahren zur Analyse des Methylierungszustands von Nukleinsäure umfassend die Schritte:
a. Bisulfitierung der Nukleinsäure;
b. Amplifikation der bisulfitierten Nukleinsäure aus Schritt a gemäß des zweiten Aspekts der Erfindung.
c. Detektion der durch die Bisulfitierung von Cytosin in Uracil umgewandelten Nukleinsäurebasen,
wobei jedes detektierte Uracil einem in der ursprünglichen Nukleinsäure vorhandenem unmethylierten Cytosin entspricht.

Ein weiterer Aspekt der vorliegenden Erfindung betrifft ein Kit aufweisend ein wie zuvor beschriebenes Reaktionsgemisch. Dieses umfasst dATP, dTTP, dCTP und dGTP sowie zumindest eine Polymerase und ist dadurch gekennzeichnet, dass die Konzentration von dATP und dTTP höher als die von dCTP und dGTP ist.

Bevorzugt handelt es sich bei der in dem erfindungsgemäßen Verfahren bzw. Reaktionsgemisch verwendeten Nukleinsäure um DNA, besonders bevorzugt um genomische DNA oder mitochondriale DNA.

In einer bevorzugten Ausführungsform entspricht die Konzentration von dATP und dTTP im Kit 200% bis 600% der Konzentration von dCTP und dGTP, vorzugsweise 300% bis 500% der Konzentration von dCTP und dGTP.

Das erfindungsgemäße Reaktionsgemisch bzw. Verfahren kann insbesondere zur Amplifikation komplexer Nukleinsäuregemische verwendet werden. Während die eingangs erwähnten Markierungsreaktionen nur bis zu einer Komplexität von rund 10³ Nukleotiden verwendbar sind, kann das erfindungsgemäße Reaktionsgemisch bzw. ein damit durchzuführendes Verfahren für zu amplifizierende Nukleinsäuren mit einer Komplexität von ≥ 10⁴ Nukleotiden, bevorzugt ≥ 10⁵, besonders bevorzugt ≥ 10⁶ und ganz besonders bevorzugt ≥ 10⁷ Nukleotiden bis maximal 10¹¹.

Weitere Ausführungsformen der Erfindung ergeben sich aus den Ansprüchen, der detaillierten Beschreibung sowie den Beispielen.

### Kurze Beschreibung der Zeichnungen

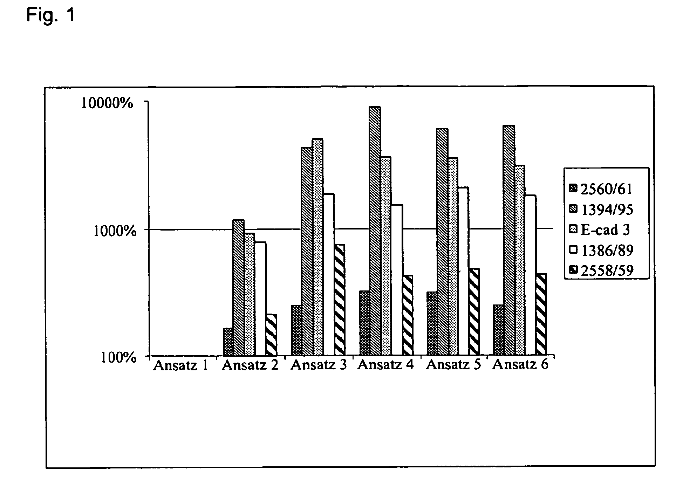

Figur 1 zeigt den Anstieg der Sequenzrepräsentanz basierend auf den mittels Real-time PCR ermittelten Ct-Werten (Tabelle 1). Eine Differenz von einem Ct-Zyklus entspricht einer 2-fachen Differenz.

### Detaillierte Beschreibung

Die Nukleinsäure-Methylierung ist ein post-replikativer, epigenetischer Mechanismus, der in Eukaryoten von erheblicher Bedeutung für die Genregulation ist. Bei der Methylierungsreaktion resultiert die Addition einer Methylgruppe an das Kohlenstoffatom Nr. 5 der Pyrimidinbase des Cytosins in der Bildung von 5-Methylcytosin. Diese Methyladdition wird *in vivo* durch einen Transfer der Methylgruppe von S-Adenosylmethionin (Methyldonor) auf Cytosin (Methylakzeptor) mithilfe von DNA-Methylasen (DNMTs) katalysiert und tritt bevorzugt bei Cytosinen auf, die 5' zu einem Guanin lokalisiert liegen (CpG).

CpG-Dinukleotide sind aufgrund evolutionärer Mechanismen und der Neigung von Methylcytosin spontan zu deaminieren im Genom unterrepräsentiert. Jedoch treten sie insbesondere in den regulatorischen Regionen von Genen als sogenannte CpG-Islands angehäuft auf, die eine Größe von ca. 500 bp - 4 kb und einen GC-Gehalt von > 55% aufweisen. Diese CpG-Islands enthalten ungefähr 20% aller CpG-Dinukleotide und sind mit den regulatorischen Regionen von ungefähr der Hälfte aller Gene des jeweiligen Genoms assoziiert. Generell liegen Gene mit hoher transkriptioneller Aktivität in unmethylierten genomischen Bereichen. Demgegenüber liegen in methylierten Bereichen Gene, die transkriptionell nur wenig oder gar nicht aktiv sind.

Da die Methylierung von DNA zu physiologischen Veränderungen wie z.B. der Verpackungsdichte von DNA, der Expression oder Inaktivierung von Genen führen kann und Veränderungen des Methylierungsmusters insbesondere mit der Entstehung von Krebs, einigen genetischen Krankheiten und der Zellalterung in Verbindung gebracht wurden, wird erwogen, die gerichtete Änderung/Korrektur des Methylierungszustands zum Zwecke der Genregulation therapeutisch einzusetzen. Insbesondere hierfür, aber auch für z.B. diagnostische Anwendungen, ist eine verlässliche Analyse des Methylierungsstatus eine unabdingbare Voraussetzung.

Für die Analyse des Methylierungzustands von Nukleinsäuren, insbesondere des Methylierungsstatus von spezifischen CpG-Stellen, hat sich die Bisulfitierung mit z.B. anschließender Amplifikation/Sequenzierung bewährt, die erstmals durch Frommer et al. (Proc Natl Acad Sci USA. 1992; 89(5):1827-31) beschrieben wurde.

Die vorliegende Erfindung stellt verbesserte Reaktionsgemische und Verfahren für die Amplifikation mittels Bisulfitierung umgewandelter Nukleinsäure, Verfahren zur Analyse des Methylierungszustands von Nukleinsäure, sowie korrespondierende Kits bereit.

### Definitionen

Der Begriff "Nukleinsäure" wie hier verwendet, bezeichnet eine Ribonukleinsäure, vorzugsweise eine Deoxyribonukleinsäure. Diese kann natürlichen Ursprungs sein, d.h. unter anderem aus Organismen, Gewebe, Zellen oder Bioptat gewonnen worden, aber auch durch andere Verfahren hergestellt worden sein, unter anderem durch *in vitro* Reaktionen enzymatischer oder chemischer Natur. Unter Nukleinsäure wird hier auch eine modifizierte Form der Nukleinsäure verstanden, die unter anderem Thioester-Verbindungen anstelle der Phosphoesther-Verbindungen, Basen- oder Deoxyribosemodifikationen wie z.B. durch Anhängen von organischen oder anorganischen Gruppen, oder eingebrachte Basenanaloga (auch non-Purin oder non-Pyrimidin Analoga) umfasst. In einer bevorzugten Ausführungsform ist die Nukleinsäure eine DNA (Deoxyribonukleinsäure), eine PNA oder eine cDNA, noch bevorzugter eine genomische oder mitochondriale DNA. Die Nukleinsäure kann einsträngig, doppelsträngig oder mehrsträngig vorliegen.

Der Begriff "umgewandelte Nukleinsäure" oder "umgewandelte DNA" wie hier verwendet, bezeichnet eine Nukleinsäure oder DNA, deren Zusammensetzung bezüglich der relativen Anteile von A, G, C oder T zueinander verändert wurde. Bevorzugt wird hierunter die Umwandlung unmethylierter Cytosine in Uracil durch Bisulfitierung der Nukleinsäure verstanden. Noch bevorzugter wird hierunter die Umwandlung der meisten oder aller unmethylierter Cytosine zu Uracil durch Bisulfitierung der Nukleinsäure verstanden, so dass mindestens 50%, bevorzugt mehr als 75%, besonders bevorzugt mehr als 90% der nicht methylierten Cytosine in Uracil umgewandelt wurden. Eine mittels Bisulfitierungsreaktion umgewandelte Nukleinsäure wird auch als "bisulfitiert" bezeichnet. Da durch die Bisulfitierung die unmethylierten Cytosine in Uracil umgewandelt werden, enthält eine umgewandelte Nukleinsäure bei ursprünglichem Vorliegen von zumindest einem unmethylierten Cytosin zumindest ein Uracil. Des Weiteren führt, da lediglich die Cytosine, nicht jedoch die Guanine des komplementären Strangs umgewandelt werden, die Umwandlung zu einer Nukleinsäure mit einem G:C-Verhältnis der komplementären Stränge von ungleich 1, d.h. die DNA entspricht nicht mehr der Chargaffschen Regel der Basenverhältnisse. Weiterhin können verschiedene Varianten einer umgewandelten Nukleinsäure vorliegen, die von der Anzahl der unmethylierten Cytosine dieser Nukleinsäure abhängen. Eine Nukleinsäure, die beispielsweise 2 Cytosine enthält kann demnach, je nach Methylierungszustand dieser Cytosine, zu 4 unterschiedlichen Varianten nach der Bisulfitierung führen, da entweder keines, das erste, das zweite, oder beide der Cytosine unmethyliert vorliegen kann/können und in Uracil umgewandelt wird/werden. Diese unterschiedlichen Varianten können mittels spezifischer Primer oder Primersätze detektiert werden. Die Umwandlung kann an gereinigter Nukleinsäure oder an Lysaten erfolgen.

Der Begriff "Amplifikation" wie hier verwendet bezeichnet die Vermehrung einer Nukleinsäure (Template- oder Matrizen-Nukleinsäure) um zumindest den Faktor 2. Bevorzugte Verfahren zur Amplifikation von Nukleinsäuren sind globale Amplifikationsverfahren, die die ganze umgewandelte Nukleinsäure einer Probe amplifizieren. Hierzu zählen isothermale und nicht-isothermale Whole-Genome-Amplification (WGA) Verfahren. Zwei für die vorliegende Erfindung besonders bevorzugte Amplifikationsverfahren sind die RCA (Rolling Circle Amplification) und die MDA (Multiple Displacement Amplification).

Die RCA basiert auf der Rolling Circle Replikation, die unter anderem bei vielen Viren stattfindet (siehe z.B. Baker, T. A. & Kornberg, A. (1992) DNA Replication; Freeman, New Y-ork). Die RCA umfasst generell mehrere Runden einer isothermal ausgeführten, enzymatisch katalysierten Nukleinsäurestrangsynthese, wobei eine Polymerase einen an eine zirkuläre Nukleinsäure hybridisierten Primer zu einem Nukleinsäurestrang verlängert, während sie kontinuierlich an der zirkulären Nukleinsäure (dem Target Circle) voranschreitet. Dieses erstmals durch Fire et al. (Proc. Natl. Acad. Sci. USA. (1995); 92(10):4641-5) beschriebene Verfahren ist durch eine lineare Kinetik, eine einfache Skalierbarkeit, die Verwendung einer Vielzahl von möglichen Polymerasen (im Gegensatz zur PCR ist keine hitzestabile Polymerase erforderlich) und einer hohen Spezifizität und Sensitivität charakterisiert und kann damit zur verlässlichen und reproduzierbaren Vervielfältigung von Nukleinsäuren bis in den Milligrammbereich genutzt werden. Die RCA kann auch zur exponentiellen Vervielfältigung von Nukleinsäure verwendet werden. Diese erreicht man mittels zumindest zwei Primern, bzw. einem Primersatz, wobei die Primer mit zumindest 2 Bindestellen auf dem Target Circle hybridisieren oder aber mit mindestens einer Bindestelle auf dem Target Circle und mindestens einer Bindestelle auf dem komplementären Strang hybridisieren. Eine Übersicht über die RCA findet sich in Demidov, V. (2005) Encyclopedia of Diagnostic Genomics and Proteomics; Marcel Dekker, Inc.; 1175-1179.

Bei der.MDA, die ebenfalls isothermal durchgeführt wird und die dieselben oben aufgeführten Vorzüge der RCA aufweist, wird eine lineare Nukleinsäure, z.B. genomische DNA, durch Hybridisierung von Primern und mittels einer Polymerase amplifiziert, wobei gleichzeitig die gebildeten Nukleinsäurestränge für ein jeweils sekundäres Hybridisieren der Primer genutzt werden, d.h. sekundäre Amplifikationsrunden an primären Amplifikationsprodukten initiiert werden. Für die RCA, sowie die MDA werden bevorzugt hochproduktive Polymerasen mit Strand-Displacement-Aktivität und mit oder ohne 3'→5' Exonuklease-Proofreading-Aktivität eingesetzt, die ohne vom Nukleinsäuretemplate abzufallen, bis zu 100 kb der Nukleinsäure mit hoher Qualität replizieren können. Ein bevorzugtes Beispiel für eine solche Polymerase ist die Phi29-DNA-Polymerase. Eine auf diese Weise durchgeführte Amplifikation hat aufgrund der Verwendung einer hitzelabilen Polymerase und der Reaktionsführung bei konstanter Temperatur mehrere Vorteile gegenüber z.B. der Amplifikation mittels PCR. So kann die verwendete Polymerase nach der Amplifikationsreaktion hitzeinaktiviert werden, um zu verhindern, dass eine Degradierung des Amplifikationsprodukts durch die Polymerase eintritt. Weiterhin wird durch die Reaktionsführung bei konstanter niedriger Temperatur kein die Amplikons enthaltendes Aerosol gebildet, das zu einer Kontamination benachbarter Reaktionen führen könnte. Bei gleichzeitiger Verwendung von Exonuklease-resistenten Zufallsprimern, ist weiterhin eine extrem gleichmäßige, von der Nukleinsäuresequenz des Templates so gut wie nicht beeinflusste Amplifikation kompletter Genome (Whole Genome Amplifikation; WGA) möglich. Eine Übersicht über die MDA und Anwendungen bei der WGA findet sich, unter anderem, in Dean et al. (Proc. Natl. Acad. Sci. USA (2002) ;99(8):5261-6).

In einer besonders bevorzugten Ausführungsform der Erfindung wird für die WGA mittels MDA das REPLI-g^{®} Kit (QIAGEN) eingesetzt, bei dem nach chemischer Denaturierung und Neutralisierung der Template-Nukleinsäure die Amplifikation in Gegenwart von Phi-29-DNA-Polymerase und Exonuklease-resistenten Zufallsprimern bei 30°C und für ungefähr 8 bis 10 Stunden oder über Nacht erfolgt. Die durchschnittliche mittels des REPLI-g^{®} Kits erzeugte Produktlänge bei einer WGA ist typischerweise größer als 10 kb und liegt in einem Bereich von ungefähr 2 kb bis 100 kb.

Weitere lineare und exponentielle für die vorliegende Erfindung geeignete Amplifikationsverfahren sind dem Fachmann geläufig, unter anderem PCR (Polymerasekettenreaktion), LCR (Ligase Chainreaction), NASBA (Nucleic Acid Sequence Based Amplification), SDA (Strand Displacement Amplification), TMA (Transcription Mediated Amplification), 3SR (Selfsustained Sequence Replication), LAMP (Loop-mediated Amplification), HDA (Helicase dependent Amplification), RPA (Recombinase-Polymerase-Amplification).

Der Begriff "Primer" wie hier verwendet, bezeichnet ein Molekül, das als Startstelle für ein Enzym mit Nukleinsäure-Polymerase- oder Nukleinsäure-Ligase-Aktivität dient. Ein Primer kann ein Protein, eine Nukleinsäure oder ein anderes Molekül sein, das sich dem Fachmann als Polymerase- oder Ligase-Startstelle geeignet erweist. Dieses Molekül kann durch intermolekulare aber auch durch intramolekulare Wechselwirkung als Startstelle dienen. Bevorzugt sind Nukleinsäure-Primer. Diese können über ihre gesamte Länge mit der Template-Nukleinsäure hybridisieren oder teilweise Misspaarungen aufweisen und weisen in der Regel eine Länge von 4 bis 30 Nukleotiden (nt) auf. Die Primer können eine Zufallssequenz oder eine degenerierte Sequenz enthalten, so dass die Primer an viele verschiedene Sequenzen der mittels Bisulfitierung umgewandelter Nukleinsäure hybridisieren kann. In anderen Fällen kann der Primer eine Sequenz enthalten, so dass der Primer nur an eine oder wenige bestimmte Sequenzen in der mittels Bisulfitierung umgewandelter Nukleinsäure hybridisieren kann. Die Länge der Primer liegt zwischen 4 und 100 nt, bevorzugt zwischen 5 und 50 nt, und ganz besonders bevorzugt zwischen 6 und 25 nt.

Der Begriff "Primersatz" wie hier verwendet, bezeichnet eine Schar von Primern, die zur Durchführung einer Amplifikation benötigt wird und besteht aus zumindest einem Primer. Für lineare Amplifikationen besteht ein Primersatz zumindest aus einem Primer. Für exponentielle Amplifikationen besteht ein Primersatz ebenfalls zumindest aus einem Primer, falls dieser an zumindest zwei unterschiedlichen Positionen der Template-Nukleinsäure hybridisiert, wodurch der zwischen den Hybridisierungspositionen liegende Nukleinsäureabschnitt exponentiell amplifiziert wird. Bevorzugt besteht der Primersatz bei exponentiellen Amplifikationen jedoch zumindest aus zwei Primern, die an zwei unterschiedlichen Positionen der Template-Nukleinsäure hybridisieren (Primerpaar); auch hier wird der zwischen den Hybridisierungspositionen liegende Nukleinsäureabschnitt exponentiell amplifiziert.

Der Begriff "korrespondierende Primersätze" wie hier verwendet, bezeichnet zumindest zwei Primersätze, die für die jeweils nicht-bisulfitierte Variante bzw. für zumindest eine bisulfitierte Variante der Nukleinsäure spezifisch sind.

Der Begriff "Polymerase" wie hier verwendet, bezeichnet ein Enzym, welches die Bildung von Phosphodiester-Bindungen zwischen einzelnen Nukleotiden innerhalb eines Nukleinsäurestrangs katalysiert (z.B. DNA- und RNA-Polymerasen). Besonders bevorzugt sind für den Einsatz bei dem erfindungsgemäßen Verfahren zur Amplifikation Polymerasen, die für Amplifikationsreaktion geeignet sind, insbesondere alle DNA-Polymerasen. Die Polymerasen lassen sich unterscheiden in hitzelabile- oder hitzestabile Enzyme. Bevorzugte Polymerasen schließen Polymerasen mit Enzym-Nummer EC 2.7.7.7, Taq-Polymerase, Polymerasen mit Proof-Reading Aktivität, Polymerasen mit Strand-Displacemenet Aktivität, mutierte Polymerase, wie auch Polymerasen mit akzessorischen Faktoren (z.B. Helicase, Einzelstrang-Bindungsproteine, Rekombinationsproteine) und Holoenzyme, die einen funktionellen DNA-Polymerase Komplex bilden, ein. In einer bevorzugten Ausführungsform ist die Polymerase eine DNA-Polymerase, noch bevorzugter eine Taq-Polymerase, eine DNA-Polymerase mit Proof-Reading Aktivität, oder eine Polymerase mit Strand-Displacement Aktivität. Eine Strand-Displacement Aktivität ist eine Eigenschaft einer Polymerase, durch welche während der Polymerase-Reaktion ein Abschälen eines "alten" Stranges ("Strand-Displacement") einer doppelsträngigen Nukleinsäure von dem anderen "alten" Strang bewirkt wird. Zu den Strand-Displacement-Polymerasen gehören alle Polymerasen, die ein Strand-Displacement durchführen können. Hierzu zählen Enzyme, wie z.B. Phi29-DNA-Polymerase, Klenow exo minus DNA-Polymerase, Vent DNA Polymerase, Deep Vent^{™} DNA Polymerase, Bst DNA Polymerase, 9oNm™ DNA Polymerase und Bca DNA Polymerase.

Die Strand-Displacement-Polymerasen können auch in mutierter Form vorliegen, z.B. als sogenannte exo minus-Varianten (d.h. ohne Exonuklease Aktivität). Hierzu zählen Polymerasen wie die Phi29-DNA-Polymerase, Klenow exo minus DNA-Polymerase. Weitere in Frage kommende Polymerasen sind Taq DNA Polymerase und Tth DNA Polymerase.

Grundsätzlich können die besagten Polymerasen auch in modifizierter Form vorliegen. Hierzu zählen trunkierte Varianten, oder solche Varianten, die gegenüber den jeweiligen Wildtypen Mutationen aufweisen. Bei letzteren kann es sich um Mutationen handeln, die die Funktionalität der Polymerasen beeinflussen, z.B. erhöhen, oder aber um Mutationen, die die Funktionalität der Polymerasen nicht beeinflussen, z.B. durch konservative Substitution.

Solche konservativen Substitutionen umfassen Variationen, bei denen eine Aminosäure durch eine andere Aminosäure aus der folgenden Gruppe ersetzt wird :

| **Grappe** | **Typ** | **Aminosäuren** |
|---|---|---|
| 1. | Kleine aliphatische, nicht-polare oder wenig polare Reste | Ala, Ser, Thr, Pro und Gly |
| 2. | Polare, negativ geladene Reste und deren Amide | Asp, Asn, Glu und Gln |
| 3. | Polare, positiv geladene Reste | His, Arg und Lys |
| 4. | Große aliphatische, nicht-polare Reste | Met, Leu, Ile, Val und Cys |
| 5. | Aromatische Reste | Phe, Tyr und Trp. |

Die folgende Liste zeigt bevorzugte konservative Substitutionen der genannten Art:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Weiterhin können die besagten Polymerasen chemisch modifiziert sein.

Besonders bevorzugt ist vorgesehen, dass die verwendete Polymerase so modifiziert ist, dass sie sogenannte "Hot start"-Eigenschaften aufweist. Eine solche Polymerase eignet sich für die Verwendung in der sogenannten "Hot start-PCR". Diese unterscheidet sich von der bekannten PCR nur im ersten Schritt. Die Polymerase-Kettenreaktion wird dabei erst gestartet, wenn das Reaktionsgemisch die gewünschte Höchsttemperatur erreicht hat. Damit erreicht man, dass die Polymerisation erst beginnt, wenn die Primer spezifisch an die DNA Sequenz gebunden haben, so dass weniger Artefakte entstehen. Dabei sind drei Wege bevorzugt, einer Polymerase "Hot start"-Eigenschaften zu verleihen:

### 1. Modifikation durch einen Antikörper:

In diesem Fall wird ein Antikörper verwendet, der reversibel an die Polymerase bindet und deren Aktivität bei normaler Umgebungstemperatur unterdrückt. Da der Antikörper anders als die Polymerase nicht thermostabil ist, denaturiert und dissoziiert er beim ersten Erhitzen. Jetzt kann die Polymerase mit ihrer Arbeit beginnen.

### 2. Chemische Modifikation:

In diesem Fall wird die Polymerase mit einem chemischen Agenz, so z.B. Formaldehyd oder einem Dicarbonsäureanhydrid, reversibel inaktiviert.

### 3. Kompetitive Hemmung:

In diesem Fall wird die Polymerase z.B. mit Polyanionen, die an das aktive Zentrum binden, kompetitiv gehemmt.

Des Weiteren können der Polymerase weitere akzessorische Faktoren zugegeben werden, die eine Amplifikation der mittels Bisulfitierung umgewandelte Nukleinsäure verbessern oder ermöglichen. Zu diesen Faktoren gehören z.B. Helicasen, Einzelstrang-DNA-Bindungsproteine (z.B. SSB, T4gp32), Rekombinationsproteine (z.B. recA, Mut Proteine).

Der Begriff "Ligase" wie hier verwendet, bezeichnet eine Ligase, die zwei NukleinsäureStränge über die Herstellung einer Phosphor-Esterbindung verknüpft (ligiert). Hierzu zählen DNA- sowie RNA-Ligasen. Somit bezeichnet "Ligase" ein Enzym mit der Enzym-Nummer EC 6.5.1.1, EC 6.5.1.2 bzw. EC 6.5.1.3.

Der Begriff "Anfangskonzentration" wie hier verwendet, bezeichnet die Konzentration der jeweiligen zu Beginn der Amplifikation der Nukleinsäure vorliegenden Substanz. Beispielsweise bezeichnet die Anfangskonzentration von dATP, dTTP, dCTP und/oder dGTP (dNTPs) die Konzentration des jeweiligen Desoxyribonukleosidtriphosphats zu Beginn der Amplifikation der umgewandelten Nukleinsäure, z.B. zu Beginn einer MDA. Da die umgewandelte Nukleinsäure, die als Template für die Amplifikation dient, eine unterschiedliche Anzahl der einzelnen Nukleotide (A, T, C, G, U) enthält, nimmt während der Amplifikation die Konzentration der dNTPs verschieden schnell, d.h. in Abhängigkeit von der Frequenz des jeweils komplementären Nukleotids der umgewandelten Nukleinsäure ab.

Der Begriff "Methylierungszustand" wie hier verwendet, bezeichnet die vorhandene oder abwesende Methylierung der einzelnen Cytosinbasen einer betrachteten Nukleinsäure. Nach der Bestimmung des Methylierungszustands einer Nukleinsäure, z.B. gemäß der Verfahren der vorliegenden Erfindung, ist daher bekannt, welche der Cytosine der betrachteten Nukleinsäure in methylierter bzw. unmethylierter Form vorlagen.

### Bisulfitierungsreaktion und Bestimmung des Methylierungstatus

Der Methylierungsstatus einer Nukleinsäure kann auf verschiedene Weisen bestimmt werden. Ein dem Fachmann hierfür als besonders geeignet bekanntes Verfahren ist die Bisulfitierung der Nukleinsäure mittels Natriumbisulfit (NaHSO₃), gefolgt von einer Amplifikation (z.B. mittels RCA oder MDA) der umgewandelten Nukleinsäure zum Zwecke der Bestimmung der umgewandelten Cytosine bzw. der nicht umgewandelten 5-Methylcytosine. Diese Bestimmung kann, unter anderem, mittels Sequenzierung erfolgen, oder aber sowohl rein qualitativ als auch quantitativ durch die Verwendung von verschiedenen Primersätzen, die für umgewandelte bzw. nicht-umgewandelte Nukleinsäureregionen spezifisch sind (komplementäre Primersätze).

Bei der von Frommer et al. (Proc Natl Acad Sci USA. 1992; 89(5):1827-31) beschriebenen Bisulfitierungsreaktion wird zu Einzelsträngen denaturierte DNA in einer Mischung von 3.1 M Natriumbisulfit und 0.5 mM Hydrochinon bei einem pH von 5.0 und bei 50° C für 16 bis 40h inkubiert, um die Umwandlung der unmethylierten Cytosine in Uracil zu erreichen. Vor der weiteren Verwendung der umgewandelten Nukleinsäure wird nach erfolgter Bisulfitierungsreaktion überschüssiges Natriumbisulfit mittels Dialyse entfernt.

Eine weitere Möglichkeit zur Durchführung der Bisulfitierungsreaktion bietet das EpiTect^{®} Bisulfitierungskit (erhältlich von QIAGEN), das eine Bisulfitierungsreaktion unter kontrollierten und eine Fragmentierung der Nukleinsäure vermeidenden Bedingungen, sowie eine bequeme Aufreinigung der umgewandelten Nukleinsäure erlaubt.

Nach der Umwandlung und der Aufreinigung der umgewandelten Nukleinsäure schließt sich im Allgemeinen wenigstens ein Amplifikationsschritt an, mittels dessen die umgewandelte Nukleinsäure vermehrt wird, um eine Detektion der umgewandelten Cytosine zu ermöglichen. In einer bevorzugten Ausführungsform erfolgt die Amplifiaktion mittels RCA oder MDA, noch bevorzugter unter Verwendung des REPLI-g^{®} Kits. Dem Fachmann sind jedoch eine Vielzahl weiterer geeigneter Amplifikations- und Detektionsverfahren bekannt. Beispielhaft wird hier auf die von Frommer et al. (Proc Natl Acad Sci U S A. 1992; 89(5): 1827-31) beschriebene Bisulfit-Genomische-Sequenzierung (BGS) oder die von Herman et al. (Proc Natl Acad Sci U S A. 1996; 93(18): 9821-26) beschriebene Methylierungs-spezifische PCR (MSP) verwiesen. Während bei der BGS die umgewandelte DNA nach Amplifikation und Klonierung in einen Vektor mittels standardisierter Verfahren sequenziert wird (z.B. Kettenabbruchverfahren), basiert die MSP auf der Bereitstellung von zumindest einem Primersatz (forward und reverse Primer), der für die methylierte und damit nicht umgewandelte, oder die unmethylierte und umgewandelte Nukleinsäuresequenz spezifisch ist. Bei Verwendung von zwei Primersätzen (jeweils spezifisch für die umgewandelte bzw. die nicht-umgewandelte Nukleinsäure) für jede interessierende Nukleinsäureposition kann dann mittels Amplifikation das Vorliegen des methylierten bzw. unmethylierten Zustands spezifisch für die interessierende Nukleinsäureposition über die Bildung eines Amplikons bestimmt werden.

### Reaktionsgemisch für die Amplifikation mittels Bisulfitierung umgewandelter Nukleinsäure, Verfahren zur Amplifikation (und Analyse des Methylierungszustands) umgewandelter Nukleinsäure

Das von der vorliegenden Erfindung bereitgestellte Reaktionsgemisch für die Nukleinsäureamplifikation beruht auf der überraschenden Entdeckung, dass die Amplifikation von Nukleinsäure, die einer Umwandlung durch eine Bisulfitierungsreaktion unterzogen wurde, durch eine Erhöhung der Konzentration der Nukleotide dATP und dTTP im Reaktionsgemisch gegenüber der Konzentration von dCTP und dGTP signifikant verbessert werden kann.

Ein erster Aspekt der vorliegenden Erfindung betrifft ein Reaktionsgemisch für die Nukleinsäureamplifikation umfassend dATP, dTTP, dCTP und dGTP, sowie Nukleinsäure enthaltend Uracil, wobei die Anfangskonzentration von dATP und dTTP höher als die von dCTP und dGTP ist. In einer Ausführungsform umfasst das Reaktionsgemisch weiterhin eine Ligase.

Ein zweiter Aspekt der vorliegenden Erfindung betrifft ein Verfahren zur Amplifikation von mittels einer Bisulfitierungsreaktion umgewandelten Nukleinsäure unter Verwendung des erfindungsgemäßen Reaktionsgemischs umfassend die Schritte:
a. In Kontakt bringen der bisulfitierten Nukleinsäure mit zumindest einem Primersatz, zumindest einer Polymerase, sowie dATP, dTTP, dCTP und dGTP, wobei die Anfangskonzentration von dATP und dTTP höher als die von dCTP und dGTP ist; und
b. Amplifikation der Nukleinsäure mittels der Polymerase.

In einer bevorzugten Ausführungsform der Erfindung erfolgt die Amplifikation mittels MDA oder RCA, noch bevorzugter unter Verwendung des REPLI-g^{®} Kits.

In einer weiteren bevorzugten Ausführungsform wird die Amplifikation der Nukleinsäure in Gegenwart einer Ligase durchgeführt.

Dem Fachmann sind die angemessenen Bedingungen zur Durchführung einer Amplifikation von Nukleinsäure, beispielsweise mittels MDA oder RCA, hinlänglich bekannt, ebenso wie die Tatsache, dass diese an die jeweiligen Eigenschaften der Nukleinsäure angepasst werden können und sollten, um eine optimale Amplifikation zu gewährleisten. So kann beispielsweise die Reaktionstemperatur während einer MDA oder RCA an die verwendeten Primerpaare und/oder Polymerase(n) angepasst werden. Ebenso kann die Annealing-Dauer und/oder - Temperatur während einer PCR in Abhängigkeit von der zu amplifizierenden Nukleinsäure variiert werden. Gleichsam können unterschiedliche Polymerasen, Puffer oder Ausgangskonzentrationen der Nukleotide oder der zu amplifizierenden Nukleinsäure eingesetzt werden.

Ein dritter Aspekt der vorliegenden Erfindung betrifft ein Verfahren zur Analyse des Methylierungszustands von Nukleinsäure umfassend die Schritte:
a. Bisulfitierung der Nukleinsäure;
b. Amplifikation der bisulfitierten Nukleinsäure aus Schritt a gemäß des zweiten Aspekts der Erfindung, d.h. unter Verwendung des erfindungsgemäßen Reaktionsgemischs.
c. Detektion der durch die Bisulfitierung von Cytosin in Uracil umgewandelten Nukleinsäurebasen,
wobei jedes detektierte Uracil einem in der ursprünglichen Nukleinsäure vorhandenem unmethylierten Cytosin entspricht.

In einer bevorzugten Ausführungsform des Reaktionsgemischs und der Verfahren der vorliegenden Erfindung ist die Anfangskonzentration von dATP gleich der von dTTP und die von dCTP gleich der von dGTP. In einer weiteren bevorzugten Ausführungsform liegt die Anfangskonzentration von dATP und dTTP bei mehr als 500% der Anfangskonzentration von dCTP und dGTP. In einer weiteren bevorzugten Ausführungsform liegt die Ausgangskonzentration von dATP und dTTP bei 200% bis 600% der Anfangskonzentration von dCTP und dGTP; noch bevorzugter liegt die Anfangskonzentration von dATP und dTTP bei 300% bis 500% der Anfangskonzentration von dCTP und dGTP. In einer weiteren bevorzugten Ausführungsform liegt die Anfangskonzentration von dATP und dTTP bei 300%, 400% oder 500% der Anfangskonzentration von dCTP und dGTP. In einer weiteren bevorzugten Ausführungsform beträgt die Anfangskonzentration von dCTP und dGTP im Reaktionsgemisch 20-3000 µM, 50-2000 µM oder 100-1000 µM.

In einer bevorzugten Ausführungsform des Reaktionsgemischs und der Verfahren der vorliegenden Erfindung ist die Polymerase eine DNA-Polymerase, noch bevorzugter eine Taq-Polymerase, eine DNA-Polymerase mit Proof-Reading Aktivität, oder eine Polymerase mit Strand-Displacement Aktivität. Noch bevorzugter ist die Polymerase ausgewählt aus der Gruppe bestehend aus Phi29-DNA-Polymerase, Klenow exo minus DNA-Polymerase, Vent^{™} DNA-Polymerase, Deep Vent^{™} DNA-Polymerase, Bst DNA-Polymerase, 9oNm™ DNA-Polymerase, Bca DNA-Polymerase und exo minus-Varianten davon. In einer weiteren bevorzugten Ausführungsform sind der Polymerase weitere akzessorische Faktoren zugegeben, die eine Amplifikation der mittels Bisulfitierung umgewandelte Nukleinsäure verbessern oder ermöglichen.

In einer weiteren Ausführungsform des Reaktionsgemischs und des zweiten Aspekts der vorliegenden Erfindung ist die Nukleinsäure eine mittels Bisulfitierung umgewandelte Nukleinsäure, die bevorzugt im Wesentlichen keine unmethylierten Cytosinbasen, noch bevorzugter keine unmethylierte Cytosinbase umfasst. Dies gilt auch für die aus Schritt a des dritten Aspekts der Erfindung resultierende Nukleinsäure. In einer weiteren Ausführungsform ist das G:C-Verhältnis der komplementären Stränge der Nukleinsäure ungleich 1 und/oder die komplementären Stränge der Nukleinsäure sind nicht vollständig komplementär zueinander. In einer bevorzugten Ausführungsform sind die komplementären Stränge der Nukleinsäure 1-10%, 10-20%, 20-30%, 30-40%, 40-50%, 50-60%, 60-70%, 70-80%, 80-90%, 90-99,9% oder zwischen (nicht einschließlich) 50 und 100%, 75 und 100% oder 90 und 100% komplementär zueinander.

In einer weiteren bevorzugten Ausführungsform des Reaktionsgemischs und der Verfahren der vorliegenden Erfindung ist die Nukleinsäure ausgewählt aus der Gruppe bestehend aus DNA, PNA, genomische DNA, mitochondriale DNA und cDNA. Noch bevorzugter ist die Nukleinsäure mitochondriale oder genomische DNA. In einer weiteren bevorzugten Ausführungsform liegt die Nukleinsäure einzelsträngig vor.

In einer weiteren Ausführungsform des Reaktionsgemischs und der Verfahren der vorliegenden Erfindung umfasst das Reaktionsgemisch weiterhin zumindest einen Primersatz. In einer bevorzugten Ausführungsform umfasst das Reaktionsgemisch zumindest zwei korrespondierende Primersätze, die für jeweils die methylierte und damit nicht-umgewandelte Variante der Nukleinsäure spezifisch sind bzw. für zumindest eine unmethylierte und damit umgewandelte Variante der Nukleinsäure. In einer weiteren bevorzugten Ausführungsform ist der zumindest eine Primersatz oder sind die zumindest zwei korrespondierende Primersätze spezifisch für zumindest eine CpG-Island-Nukleinsäuresequenz und/oder liegt/liegen in zumindest einer regulatorischen Region zumindest eines Gens (z.B. einem Promotor).

CpG-Islands sind genomische Regionen mit statistisch erhöhter CpG-Dinukleotid-Dichte. Diese Dichte wird auf die Einzelnukleotid- und Dinukleotidfrequenzen im gesamten betrachteten Genomausschnitt bezogen. CpG bedeutet Cytosin-phosphatidyl-Guanosin. Das p wird mit angegeben, um GC von CG innerhalb von Phosphonukleosidketten (=DNA-Molekülen) besser zu unterscheiden (siehe auch 5'-Ende, 3'-Ende). CpG-Islands sind definiert als DNA-Abschnitte von 0,5kb bis 2kb Länge, die einen erhöhten GC-Gehalt von über 60% aufweisen, dabei liegt der GC-Gehalt des Gesamtgenoms bei 40%. CpG-Islands entstehen durch Mechanismen, die mit der Nutzung der Erbsubstanz als Informationsträger zu tun haben. Dadurch sind CpG-Islands wichtige Markierungen, die z. B. für die Genetik, Medizin und Bioinformatik Bedeutung haben.

In einer weiteren bevorzugten Ausführungsform ist der zumindest eine Primersatz oder sind die zumindest zwei korrespondierende Primersätze spezifisch für zumindest eine Nukleinsäuresequenz umfassend zumindest ein CpG-Island. In einer weiteren bevorzugten Ausführungsform ist der zumindest eine Primersatz oder sind die zumindest zwei korrespondierende Primersätze spezifisch für zumindest eine CpG-Island Nukleinsäuresequenz, deren Methylierungszustand mit einem Phänotyp in Verbindung gebracht wird. Vorzugsweise umfasst das Reaktionsgemisch zwischen 5 und 10, 10 und 20, 20 und 50, 50 und 100, 100 und 250, oder mehr als 250 Primersätze, korrespondierende Primersätze, oder Zufallsprimer. In einer weiteren bevorzugten Ausführungsform ist zumindest einer, vorzugsweise die Hälfte, noch bevorzugter alle dieser Primersätze spezifisch für eine CpG-Island Nukleinsäuresequenz, deren Methylierungszustand mit einer Krankheit in Verbindung gebracht wird.

In einer weiteren Ausführungsform des Reaktionsgemischs und der Verfahren der vorliegenden Erfindung umfasst das Reaktionsgemisch weiterhin einen Amplifikationspuffer. Die Zusammensetzungen solcher Puffer sind dem Fachmann hinlänglich bekannt.

In einer weiteren Ausführungsform der erfindungsgemäßen Verfahren erfolgt vor der Bisulfitierungsreaktion ein zusätzlicher Denaturierungsschritt der Nukleinsäure. In einer bevorzugten Ausführungsform erfolgt die Denaturierung reversibel und/oder thermisch und/oder unter alkalischen Bedingungen. In einer weiteren Ausführungsform erfolgt nach der Bisulfitierungsreaktion ein zusätzlicher Aufreinigungsschritt der umgewandelten Nukleinsäure. In einer bevorzugten Ausführungsform erfolgt diese Aufreinigung mittels Dialyse oder Präzipitation. In einer weiteren Ausführungsform erfolgt die Amplifikation der Nukleinsäure linear oder exponentiell, bevorzugt exponentiell.

In einer weiteren Ausführungsform des erfindungsgemäßen Verfahren zur Analyse des Methylierungszustands von Nukleinsäure erfolgt die Bisulfitierung der Nukleinsäure unter Verwendung des oben beschriebenen Verfahrens nach Frommer *et al*., bevorzugt unter Verwendung des QIAGEN EpiTect^{®} Kits. In einer weiteren Ausführungsform erfolgt die Detektion mittels Gelauftrennung, Southern-Blotting, Sequenzierung oder RT-PCR.

### Kits

Die vorliegende Erfindung stellt weiterhin Kits zur verbesserten Amplifikation von mittels Bisulfitierung umgewandelter Nukleinsäure, sowie zur Bestimmung des Methylierungszustands von Nukleinsäure bereit.

In einer Ausführungsform umfasst das erfindungsgemäße Kit dATP, dTTP, dCTP, dGTP und zumindest eine Polymerase, wobei die Konzentration von dATP und dTTP höher als die von dCTP und dGTP ist. In einer bevorzugten Ausführungsform beträgt die Konzentration von dATP und dTTP 200% bis 600% der Konzentration von dCTP und dGTP, vorzugsweise 300% bis 500% der Konzentration von dCTP und dGTP. In einer weiteren Ausführungsform liegen dCTP und dGTP in einer Konzentration von 10 mM vor. In einer weiteren Ausführungsform liegen die Nukleotide in lyophylisierter Form vor, die nach Rekonstituierung mit Lösungsmitteln, z.B. Wasser, die erfindungsgemäßen Konzentrationen und Konzentrationsverhältnisse ergibt.

In einer weiteren Ausführungsform umfasst das erfindungsgemäße Kit weiterhin zumindest einen Primersatz. In einer bevorzugten Ausführungsform umfasst das Kit zumindest zwei korrespondierende Primersätze, die für jeweils die methylierte und damit nicht-umgewandelte Variante der Nukleinsäure spezifisch sind bzw. für zumindest eine unmethylierte und damit umgewandelte Variante der Nukleinsäure. In einer weiteren bevorzugten Ausführungsform ist der zumindest eine Primersatz oder sind die zumindest zwei korrespondierende Primersätze spezifisch für zumindest eine CpG-Island-Nukleinsäuresequenz und/oder liegt/liegen in zumindest einer regulatorischen Region zumindest eines Gens (z.B. einem Promotor). In einer weiteren bevorzugten Ausführungsform ist der zumindest eine Primersatz oder sind die zumindest zwei korrespondierende Primersätze spezifisch für zumindest eine Nukleinsäuresequenz umfassend zumindest ein CpG-Island. In einer weiteren bevorzugten Ausführungsform ist der zumindest eine Primersatz oder sind die zumindest zwei korrespondierende Primersätze spezifisch für zumindest eine CpG-Island Nukleinsäuresequenz, deren Methylierungszustand mit einem Phänotyp in Verbindung gebracht wird. Vorzugsweise umfasst das Reaktionsgemisch zwischen 5 und 10, 10 und 20, 20 und 50, 50 und 100, 100 und 250, oder mehr als 250 Primersätze, korrespondierende Primersätze, oder Zufallsprimer. In einer weiteren bevorzugten Ausführungsform ist zumindest einer, vorzugsweise die Hälfte, noch bevorzugter alle dieser Primersätze spezifisch für eine CpG-Island Nukleinsäuresequenz, deren Methylierungszustand mit einer Krankheit in Verbindung gebracht wird. In einer weiteren Ausfiihrungsform umfasst das erfindungsgemäße Kit eine Polymerase in Gegenwart einer Ligase.

In einer bevorzugten Ausführungsform ist die Polymerase eine DNA-Polymerase, noch bevorzugter eine Taq-Polymerase, eine DNA-Polymerase mit Proof-Reading Aktivität, oder eine Polymerase mit Strand-Displacement Aktivität. Noch bevorzugter ist die Polymerase ausgewählt aus der Gruppe bestehend aus Phi29-DNA-Polymerase, Klenow exo minus DNA-Polymerase, Vent^{™} DNA-Polymerase, Deep Vent^{™} DNA-Polymerase, Bst DNA-Polymerase, 9oNm™ DNA-Polymerase, Bca DNA-Polymerase und exo minus-Varianten davon. In einer weiteren bevorzugten Ausführungsform sind der Polymerase weitere akzessorische Faktoren zugegeben, die eine Amplifikation der mittels Bisulfitierung umgewandelte Nukleinsäure verbessern oder ermöglichen.

### Beispiele

### Beispiel 1:

Genomische DNA wurde mit Hilfe der QIAamp Methode (QIAGEN) nach Standardprotokoll aus humanem Blut gewonnen. 1 µg der so gewonnenen DNA wurde für eine Bisulfitierungsreaktion (Umwandlungsreaktion) mit dem EpiTect Kit (QIAGEN) nach Standardprotokoll eingesetzt. 5 µl der gereinigten umgewandelten DNA wurde für eine Whole-Genome-Amplification verwendet. Hierzu wurde das REPLI-g Kit (QIAGEN) eingesetzt, wobei zu 5 µl der gereinigten umgewandelten DNA 29 µl des REPLI-g Midi Reaction Buffers, 2 µl der REPLI-g Midi-Polymerase (Phi29-Polymerase) und unterschiedliche Volumina eines Mixes von 10 mM dATP und dTTP hinzugefügt wurde. Die Anfangskonzentration von dGTP und dCTP wurde konstant auf 1 mM festgesetzt.

**Tabelle 1: Volumina des dATP/dTTP Mixes, die den REPLI-g Reaktionen zugesetzt wurden und die sich daraus ergebenden Anfangskonzentrationen im Ansatz**

| | | **dATP** | **dTTP** | **dGTP** | **dCTP** |
|---|---|---|---|---|---|
| Ansatz 1 | 0 µl dATP/dTTP | 1 mM | 1 mM | 1 mM | 1 mM |
| Ansatz 2 | 1 µl dATP/dTTP | 1,24 mM | 1,24 mM | 1 mM | 1 mM |
| Ansatz 3 | 2 µl dATP/dTTP | 1,48 mM | 1,48 mM | 1 mM | 1 mM |
| Ansatz 4 | 3 µl dATP/dTTP | 1,73 mM | 1,73 mM | 1 mM | 1 mM |
| Ansatz 5 | 4 µl dATP/dTTP | 1,97 mM | 1,97 mM | 1 mM | 1 mM |
| Ansatz 6 | 5 µl dATP/dTTP | 2,22 mM | 2,22 mM | 1 mM | 1 mM |

Der Reaktionsansatz hatte ein Gesamtvolumen von 41 µl. Die Reaktion wurde bei 30°C für 8 Stunden inkubiert. Nach der Inkubation wurde die Reaktion zum Stoppen für 5 min bei 65°C inkubiert. Die entstandene DNA wurde mittels PicoGreen (Invitrogen) quantifiziert. 10 ng der amplifizierten DNA wurde mittels Real-time PCR auf bisulfitierte Genomsequenzen hin untersucht. Die Real-time PCR wurde mittels QuantiTect SybrGreen Reagenzien (QIAGEN) nach Standardprotokoll durchgeführt.

Folgende Primerpaare wurden für die Real-time PCR mit dem QuantiTect SybrGreen Kit (QIAGEN) verwendet:

| | |
|---|---|
| 2560/61: | GTGATGGTGGGTATGGGTTAGAAGGATTTT und |
| | CAACTCATTATAAAAAATATAATACCAAA |
| 1393/94: | CCCCTTCTAAAAACTCCCCAA und |
| | TGTAGGGGAATTGGAATTAGGT |
| Ecad3: | TGGTTGTAGTTATGTATTTATTTTTAGTGGTGTT UND |
| | ACACCAAATACAATCAAATCAAACCAAA |
| 1386/89: | GAGAGAGAAGTAGTTGTGTA und |
| | CCATTCTATCTCCAATAACACCCT |
| 2558/59: | GGATTTGATTGATTATTTTATGAAGATTTTTAT und |
| | CCATACCCAAAAAAAAAAACTAAAAAAATACC |

Tabelle 2 zeigt die für die verschiedenen Primerpaare mittels der Real-time PCR ermittelten Ct-Werte. Der Ct-Wert (Threshold Cycle) entspricht den Zyklus der PCR, an dem die Fluoreszenz erstmalig signifikant über die Hintergrund-Fluoreszenz ansteigt. Somit entspricht, bei gleicher Ausgangsmenge an Template-DNA, ein niedrigerer Ct-Wert einer effektiver verlaufenden PCR.

**Tabelle 2: Mittels Real-time PCR ermittelte Ct-Werte**

| | **2560/61** | **1393/94** | **E-cad 3** | **1386/59** | **2558/59** |
|---|---|---|---|---|---|
| **Ansatz 1** | 28,89 | 31,07 | 31,65 | 32,40 | 34,26 |
| **Ansatz 2** | 28,2 | 27,50 | 28,4 | 29,42 | 33,18 |
| **Ansatz 3** | 27,58 | 25,65 | 26,00 | 28,18 | 31,36 |
| **Ansatz 4** | 27,19 | 24,59 | 26,47 | 28,46 | 32,16 |
| **Ansatz 5** | 27,2 | 25,16 | 26,5 | 28,01 | 31,99 |
| **Ansatz 6** | 27,59 | 25,07 | 26,70 | 28,22 | 32,14 |

Wie den Resultaten zu entnehmen ist, werden die Ct-Werte bei einer Amplifikation von Nukleinsäure, die einer Umwandlung durch eine Bisulfitierungsreaktion unterzogen wurde, durch eine Erhöhung der Anfangskonzentration der Nukleotide dATP und dTTP im Reaktionsgemisch gegenüber der Anfangskonzentration von dCTP und dGTP signifikant erniedrigt, was einer verbessert bzw. effektiver verlaufenden PCR entspricht. Dies gilt für alle getesteten Primerkombinationen und ist insbesondere bei Ansätzen 3 bis 6 der Fall, d.h. bei einer Erhöhung der Anfangskonzentration der Nukleotide dATP und dTTP im Reaktionsgemisch gegenüber der Anfangskonzentration von dCTP und dGTP von ungefähr 50%-120%.

Figur 1 zeigt den Anstieg der Sequenzrepräsentanz basierend auf den ermittelten Ct-Werten, wobei eine Differenz von einem Ct-Zyklus einer 2-fachen Differenz entspricht. Auch hier ist deutlich zu erkennen, dass für alle getesteten Primerkombinationen die Erhöhung der Anfangskonzentration der Nukleotide dATP und dTTP im Reaktionsgemisch gegenüber der Anfangskonzentration von dCTP und dGTP zu einer signifikanten Verbesserung der Amplifikation der bisulfitierten Nukleinsäure führte.

### Beispiel 2:

Genomische DNA wurde mit Hilfe der QIAamp Methode (QIAGEN) nach Standardprotokoll aus humanem Blut gewonnen. 1 µg der so gewonnenen DNA wurde für eine Bisulfitierungsreaktion (Umwandlungsreaktion) mit dem EpiTect Kit (QIAGEN) nach Standardprotokoll eingesetzt. 5 µl der gereinigten umgewandelten DNA wurde für eine Whole-Genome-Amplification verwendet. Hierzu wurde das REPLI-g Kit (QIAGEN) eingesetzt, wobei zu 5 µl der gereinigten umgewandelten DNA 29 µl des REPLI-g Mini Reaction Buffers, 1 µl der REPLI-g Midi-Polymerase (Phi29-Polymerase) und unterschiedliche Volumina eines Mixes von 5 mM dATP und dTTP hinzugefügt wurde. Die Anfangskonzentration von dGTP und dCTP wurde konstant auf 0,24 mM festgesetzt.

**Tabelle 3: Volumina des dATP/dTTP Mixes, die den REPLI-g Reaktionen zugesetzt wurden und die sich daraus ergebenden Anfangskonzentrationen im Ansatz**

| | | **dATP** | **dTTP** | **dGTP** | **dCTP** |
|---|---|---|---|---|---|
| Ansatz 1 | 0 µl dATP/dTTP | 0,24 mM | 0,24 mM | 0,24 mM | 0,24 mM |
| Ansatz 2 | 5 µl dATP/dTTP | 0,83 mM | 0,83 mM | 0,24 mM | 0,24 mM |
| Ansatz 3 | 6 µl dATP/dTTP | 0,95 mM | 0,95 mM | 0,24 mM | 0,24 mM |
| Ansatz 4 | 7 µl dATP/dTTP | 1,07 mM | 1,07 mM | 0,24 mM | 0,24 mM |
| Ansatz 5 | 8 µl dATP/dTTP | 1,19 mM | 1,19 mM | 0,24 mM | 0,24 mM |
| Ansatz 6 | 9 µl dATP/dTTP | 1,31 mM | 1,31 mM | 0,24 mM | 0,24 mM |

Der Reaktionsansatz hatte ein Gesamtvolumen von 41 µl. Die Reaktion wurde bei 30°C für 8 Stunden inkubiert. Nach der Inkubation wurde die Reaktion zum Stoppen für 5 min bei 65°C inkubiert. Die entstandene DNA wurde mittels PicoGreen (Invitrogen) quantifiziert. 10 ng der amplifizierten DNA wurde mittels Real-time PCR auf bisulfitierte Genomsequenzen hin untersucht. Die Real-time PCR wurde mittels QuantiTect SybrGreen Reagenzien (QIAGEN) nach Standardprotokoll durchgeführt.

Folgende Primerpaare wurden für die Real-time PCR mit dem QuantiTect SybrGreen Kit (QIAGEN) verwendet:

| | |
|---|---|
| 699bis: | GGTAGGGATTTGTGATATTGT und |
| | AATACCATAACACATAACCTAA |
| 1386/89: | GAGAGAGAAGTAGTTGTGTA und |
| | CCATTCTATCTCCAATAACACCCT |
| C3: | GGAGTGGAGGAAATTGAGAT UND |
| | CCACACAACAAATACTCAAAAC |

Tabelle 4 zeigt die für die verschiedenen Primerpaare mittels der Real-time PCR ermittelten Ct-Werte. Der Ct-Wert (Threshold Cycle) entspricht den Zyklus der PCR, an dem die Fluoreszenz erstmalig signifikant über die Hintergrund-Fluoreszenz ansteigt. Somit entspricht, bei gleicher Ausgangsmenge an Template-DNA, ein niedrigerer Ct-Wert einer effektiver verlaufenden PCR.

**Tabelle 4: Mittels Real-time PCR ermittelte Ct-Werte**

| | **699bis** | **1386/89** | **C 3** |
|---|---|---|---|
| **Ansatz 1** | 30,21 | 30,76 | 31,43 |
| **Ansatz 2** | 27,41 | 28,11 | 28,47 |
| **Ansatz 3** | 26,35 | 29,34 | 28,90 |
| **Ansatz 4** | 26,98 | 28,87 | 29,01 |
| **Ansatz 5** | 25,54 | 27,92 | 28,04 |
| **Ansatz 6** | 26,18 | 29,34 | 28,17 |

Wie den Resultaten zu entnehmen ist, werden die Ct-Werte bei einer Amplifikation von Nukleinsäure, die einer Umwandlung durch eine Bisulfitierungsreaktion unterzogen wurde, durch eine Erhöhung der Anfangskonzentration der Nukleotide dATP und dTTP im Reaktionsgemisch gegenüber der Anfangskonzentration von dCTP und dGTP signifikant erniedrigt, was einer verbessert bzw. effektiver verlaufenden PCR entspricht. Dies gilt für alle getesteten Primerkombinationen und ist insbesondere bei Ansätzen 2 bis 6 der Fall, d.h. bei einer Erhöhung der Anfangskonzentration der Nukleotide dATP und dTTP im Reaktionsgemisch gegenüber der Anfangskonzentration von dCTP und dGTP von ungefähr 350%-550%.

## Patentansprüche

1. Reakrionsgemisch zur Amplifikation von bisulfitierten Nukleinsäuren umfassend dATP, dTTP, dCTP und dGTP, sowie bisulfitierten Nukleinsäure, enthaltend Uracil, **dadurch gekennzeichnet, dass** die molare Anfangskonzentration von dATP und dCIP 200% bis 600% höher ist als die molare Anfangskonzentration von dCTP und dGTP und vorzugsweise 300% bis 500% der molaren Anfangskonzentration von dCTP und dGTP beträgt.

2. Reaktionsgemisch gemäß Anspruch 1, mit zümindest einen Primersatz für die jeweils nicht-bisulfitierte Variante und/oder für zumindest eine bisulfitierte Variante der Nukleinsäure spezifisch ist.

3. Reaktionsgemisch gemäß einem der vorstehenden Ansprüche, wobei die Ausgangskonzentration von dCTP und dGTP 20-3000 µM, 650-2000 µM oder 100-1000 µM ist.

4. Reaktionsgemisch gemäß einem der vorstehenden Ansprüche, weiterhin umfassend zumindest eine Polymerase.

5. Reaktionsgemisch gemäß Anspruch 4, wobei die Polymerase eine Strand-Displacement-Polymerase oder eine hitzestabile Polymerase ist.

6. Verfahren zur Amplifikation von mittels Bisulfitierungsreaktion umgewandelter Nukleinsäure umfassend die Schritte:
a) In Kontakt bringen der bisulfitierten Nukleinsäure mit zumindest einem Primersatz, zumindest einer Polymerase, sowie dATP, dTTP, dCTP und dGTP; und
b) Amplifikation der Nukleinsäure mittels der zumindest einen Polymerase,
**dadurch gekennzeichnet, dass** die Anfangskonzentration von dATP und dTTP 200% bis 600% der molaren Anfangskonzentration von dCTP und dGTP, vorzugsweise 300% bis 500% der molaren Anfangskonzentration von dCTP und dGTP beträgt.

7. Verfahren gemäß Anspruch molaren 6, wobei die Nukleinsäure mit zumindest zwei korrespondierende Primersätzen in Kontakt gebracht wird, wobei die korrespondierenden Primersätze für die jeweils nicht-bisulfitierte Variante bzw. für zumindest eine bisufitierte Variante der Nukleinsäure spezifisch sind.

8. Verfahren zur Analyse des Methylierungszustands von Nukleinsäuren, umfassend die Schritte:
a) Bisulfitierung der Nukleinsäure;
b) Amplifikation der bisulfitierten Nukleinsäure aus Schritt a gemäß einem der Ansprüche 6 - 7; und
c) Detektion der durch die Bisulfitierung von Cytosin in Uracil umgewandelten Nukleinsäurebasen,
wobei jedes detektierte Uracil einem in der ursprünglichen Nukleinsäure vorhandenem unmethylierten Cytosin entspricht.

9. Reaktionsgemisch gemäß einem der Ansprüche 1 - 5, wobei es sich bei der Nukleinsäure um DNA handelt.

10. Verfahren gemäß einem der Ansprüche 6-8, wobei es sich bei der Nukleinsäure um DNA handelt.

11. Kit aufweisend ein Reaktionsgemisch gemäß einem der Ansprüche 1 - 5 und/oder 9, besagtes Kit weiterhin umfassend eine Ligase.

12. Kit gemäß Anspruch 11, weiterhin umfassend zumindest einen Primersatz.

13. Kit gemäß Anspruch 12, umfassend zumindest zwei, zwischen 5 und 10, 10 und 20, 20 und 50, 50 und 100, 100 und 250, oder mehr als 250 korrespondierende Primersätze, wobei die korrespondierenden Primersätze für die jeweils nicht-bisulfitierte Variante bzw. für zumindest eine bisulfitierte Variante einer Nukleinsäure spezifisch sind.

14. Kit gemäß Anspruch 13, wobei zumindest ein Primersatz für zumindest eine CpG-Island Nukleinsäuresequenz spezifisch ist, deren Methytierungszustand mit einem Phänotyp oder einer Krankheit in Verbindung gebracht wird.

## Claims

1. Reaction mixture for the amplification of bisulfite-reacted nucleic acids comprising dATP, dTTP, dCTP and dGTP, and bisulfite-reacted nucleic acids comprising uracil **characterized in that** the dATP and dTTP initial molar concentration is 200% to 600% higher compared to the dCTP and dGTP initial molar concentration and preferably 300% to 500% higher compared to the dCTP and dGTP initial molar concentration.

2. The reaction mixture of claim 1, wherein at least one set of primers is specific to the non bisulfite-reacted variant and/or is specific to at least one bisulfite-reacted nucleic acid variant respectively.

3. The reaction mixture of any of the preceding claims, wherein the dCTP and dGTP initial concentration is 20-3000 µM, 650-2000 µM or 100-1000 µM.

4. The reaction mixture of any of the preceding claims additionally comprising at least one polymerase.

5. The reaction mixture according to claim 4, wherein the polymerase is a strand-displacement- or a heat-stabilized-polymerase.

6. A method for amplification ofbisulfite-reacted nucleic acids comprising the steps of
a) Contacting the bisulfite-reacted nucleic acid and at least one primer-set, at least one polymerase and dATP, dTTP, dCTP and dGTP; and
b) Amplifying the nucleic acid using at least said polymerase, **characterized in that** the initial molar dATP and dTTp concentration is 200% to 600%, preferably 300% to 500% of the dCTP and dGTP initial molar concentration.

7. A method according to claim 6, wherein the nucleic acid is contacted with at least two corresponding primer-sets, wherein the corresponding primer-sets are each specific to the non-bisulfite reacted variant or alternatively to the at least one bisulfite-reacted nucleic acid variant.

8. A method to analyze the methylation state of nucleic acids, comprising the steps of:
a) Reacting the nucleic acid with bisulfite;
b) Amplifying the bisulfite-reacted nucleic acid of step a) according to any of the claims 6-7; and
c) Detecting the Uracil nucleic acid bases, which has been generated from Cytosin by bisulfite-modifaction,
wherein each detected Uracil corresponds in the original nucleic acid to a non-methylated Cytosin.

9. The reaction mixture according to any of the claims 1 to 5, wherein the nucleic acid is DNA.

10. A method according to any of the claims 6-8, wherein the nucleic acid is DNA.

11. A Kit comprising a reaction mixture of any of the claims 1 - 5 and/or claim 9, wherein the kit further comprises a ligase.

12. A Kit according to claim 11, further comprising at least one primer-set.

13. A Kit according to claim 12, comprising at least two, between 5 to 10, 10 to 20, 20 to 50, 50 to 100, 100 to 250, or more than 250 corresponding primer-sets, wherein the corresponding primer-sets are specific to the non-bisulfite reacted variant or alternatively are specific to at least one bisulfite-reacted variant of a nucleic acid.

14. A Kit according to claim 13, wherein at least one primer-set is specific to the CpG-Island nucleic acid sequence, which methylation state can be linked to a phenotype or illness.

## Revendications

1. Mélange réactionnel pour l'amplification d'acides nucléiques bisulfités, comprenant dATP, dTTP, dCTP et dGTP, ainsi que des acides nucléiques bisulfités, contenant de l'uracile, **caractérisé en ce que** la concentration molaire initiale de dATP et de dTTP est 200 % à 600 % supérieure à la concentration molaire initiale de dCTP et dGTP et vaut de préférence 300 % à 500 % de la concentration molaire initiale de dCTP et de dGTP.

2. Mélange réactionnel selon la revendication 1, avec au moins un ensemble d'amorces pour la variante non bisulfitée respective et/ou pour au moins une variante bisulfitée de l'acide nucléique.

3. Mélange réactionnel selon l'une des revendications précédentes, la concentration initiale de dCTP et de dGTP étant de 20 à 3 000 µM, de 650 à 2 000 µM ou de 100 à 1 000 µM.

4. Mélange réactionnel selon l'une des revendications précédentes, comprenant en outre au moins une polymérase.

5. Mélange réactionnel selon la revendication 4, la polymérase étant une polymérase à déplacement de brin ou une polymérase thermostable.

6. Procédé pour l'amplification d'un acide nucléique transformé au moyen d'une réaction de bisulfitation, comprenant les étapes :
a) mise en contact de l'acide nucléique bisulfité avec au moins un ensemble d'amorces, à savoir au moins une polymérase, ainsi que dATP, dTTP, dCTP et dGTP ; et
b) amplification de l'acide nucléique au moyen de la ou des polymérases ; **caractérisé en ce que** la concentration initiale de dATP et de dTTP vaut 200 % à 600 % de la concentration molaire initiale de dCTP et de dGTP, de préférence 300 % à 500 % de la concentration molaire initiale de dCTP et de dGTP.

7. Procédé selon la revendication 6, l'acide nucléique étant amené en contact avec au moins deux ensembles d'amorces correspondants, les ensembles d'amorces correspondants étant spécifiques à la variante non bisulfitée respective ou à au moins une variante bisulfitée de l'acide nucléique.

8. Procédé pour l'analyse de l'état de méthylation d'acides nucléiques, comprenant les étapes :
a) bisulfitation de l'acide nucléique ;
b) amplification de l'acide nucléique bisulfité issu de l'étape a) selon l'une des revendications 6 ou 7 ; et
c) détection des bases d'acide nucléique transformées par la bisulfitation de cytosine en uracile ;
chaque uracile détecté correspondant à une cytosine non méthylée présente dans l'acide nucléique initial.

9. Mélange réactionnel selon l'une des revendications 1 à 5, l'acide nucléique étant un ADN.

10. Procédé selon l'une des revendications 6 à 8, l'acide nucléique étant un ADN.

11. Kit comportant un mélange réactionnel selon l'une des revendications 1 à 5 et/ou 9, ledit kit comportant en outre une ligase.

12. Kit selon la revendication 11, comprenant aussi au moins un ensemble d'amorces.

13. Kit selon la revendication 12, comprenant au moins deux, entre 5 et 10, 10 et 20, 20 et 50, 50 et 100, 100 et 250, ou plus de 250, ensembles d'amorces correspondants, les ensembles d'amorces correspondants étant spécifiques à la variante non bisulfitée respective ou à au moins une variante bisulfitée d'un acide nucléique.

14. Kit selon la revendication 13, au moins un ensemble d'amorces étant spécifique à au moins une séquence d'acide nucléique à îlot CpG dont l'état de méthylation est mis en relation avec un phénotype ou avec une maladie.
